# EUROPEAN PATENT APPLICATION

(11) **EP 4 375 379 A1**
(43) Date of publication of application: **29.05.2024**
(21) Application number: 22306737.2
(22) Date of filing: 24.11.2022
(51) Int. Cl.: C12P 7/42, C12N 15/70

(54) **BIOPRODUCTION OF GLYCOLIC ACID FROM GLYOXAL**

(71) Applicant: BIOC3, 31077 Toulouse CEDEX 4 (FR)
(72) Inventor: LACHAUX, Cléa, 31500 TOULOUSE (FR); GRONDIN, Eric, 31320 CASTANET TOLOSAN (FR)
(74) Representative: Regimbeau

(57) **Abstract**

The invention relates to a method for the biochemical production of glycolic acid or its derivatives comprising a step of culturing a whole cell biocatalyst comprising or consisting of modified microorganisms cells producing an enzyme converting glyoxal into glycolic acid, in a suitable culture medium comprising glyoxal as substrate, and optionally recovering glycolic acid from the culture medium.

## Description

### FIELD OF THE INVENTION

The present invention belongs to the field of the production of molecules of interest by engineered microorganisms, and relates more particularly to the bioconversion of glyoxal into glycolic acid, by enzymatic catalysis carried out in whole cells of microorganisms.

### BACKGROUND OF THE INVENTION

Glycolic acid (HOCH2COOH) belongs to the family of α-hydroxy acids, it is a two-carbon organic acid with an alcohol function and a carboxylic function. In monomeric form, glycolic acid is mainly used in cosmetics and cleaning products. In polymerized form, it forms a polymer called polyglycolate, which exhibits remarkable mechanical properties suitable for packaging.

Commercially available glycolic acid is generally produced by plant extraction and chemical synthesis from 3 processes:
- Carbonylation of formaldehyde with carbon monoxide at high temperature (200-250 C) and high pressure (300-700 bar) in the presence of an acid catalyst (H2SO4). But the final product is colored by high temperature oxidation and contains traces of formaldehyde, which is toxic, carcinogenic, potentially mutagenic, and is subject to restrictions and prohibitions in the field of cosmetics (European regulation (CE) N°1223/2009;
- Neutralization and reacidification of monochloroacetic acid (MCA). MCA is produced from chlorine gas and acetic acid. But the final product contains salts and chlorinated organic compounds;
- Enzymatic conversion of glyconitrile to glycolic acid by nitrilases. But the main disadvantage of this process is the presence of glyconitrile which is listed as an extremely hazardous substance, as it can polymerize under the influence of traces of acid or base, with a risk of fire or explosion.

Several international publications also report bioproduction of glycolic acid from ethylene glycol using yeasts or bacteria that naturally convert ethylene glycol into glycolic acid, as this process has the advantage of operating under physiological conditions. Ethylene glycol is inexpensive but the bioproduction by fermentation is generally too long (2 to 5 days) for industrial application.

Metabolic engineering has made it possible to exceed the production capacities of organisms that naturally produce glycolic acid from ethylene glycol, but there is still a need to provide new bioproduction method of glycolic acid under mild conditions (25°C, pH 7.0) and with improved performance.

The present invention specifically proposes a new method of producing glycolic acid involving a microbial strain modified to express a glyoxalase, either in the cytoplasm or in the periplasm of the cell. In particular, the "biocatalyst" is a strain of *Escherichia coli (E.coli)* expressing a glyoxalase that effectively catalyzes the bioconversion of glyoxal to glycolic acid under mild conditions (25°C, 1 atm, pH 7.5).

### SUMMARY OF THE INVENTION

A first object of the present invention is a method for the biochemical production of glycolic acid or its derivatives comprising a step of culturing a whole cell biocatalyst comprising or consisting of modified microorganisms cells producing an enzyme converting glyoxal into glycolic acid, in a suitable culture medium comprising glyoxal as substrate, and optionally recovering glycolic acid from the culture medium.

Another object of the present invention is a whole cell biocatalyst comprising or consisting of modified microorganisms cells producing a enzyme able to convert glyoxal into glycolic acid, as defined in the present invention.

The present invention also relates to a method of producing a whole cell biocatalyst as defined in the present invention, comprising the steps of:
a) Transforming the microorganisms cells by a plasmid comprising an expression cassette comprising a gene encoding the enzyme converting glyoxal into glycolic acid (i.e glyoxalase) under the control of a constitutive or inducible promoter, and comprising optionally a secretion signal gene for addressing the enzyme into the periplasmic space; or alternatively a') engineering the microorganisms cells by integrating the said expression cassette into their genome;
b) Cultivating the modified cells in a suitable culture medium for the expression of the said enzyme (glyoxalase);
c) Separating the cells from the supernatant by centrifugation; and
d) Optionally drying the cells and storing them at 4°C or -20°C or -80°C.

Another object of the present invention is a method of producing a glycolic acid derivative or a product obtained by a reaction using glycolic acid as substrate, comprising at least a step of the method for the biochemical production of glycolic acid as defined in the present invention.

The present invention also relates to an expression cassette comprising a nucleotidic sequence encoding the enzyme converting glyoxal into glycolic acid (i.e glyoxalase) and optionally a secretion signal gene encoding a signal peptide as defined in the invention for adressing the said enzyme (i.e glyoxalase) into the periplasmic space of a microorganism cell.

Another object of the present invention relates to a nucleotide sequence encoding the polypeptide or expression cassette as defined in the invention.

The present invention also concerns a vector comprising a nucleotide sequence as defined in the invention.

### DETAILED DESCRIPTION OF THE INVENTION

So, a first object of the present invention is a method for the biochemical production of glycolic acid or its derivatives comprising a step of culturing a whole cell biocatalyst comprising or consisting of modified microorganisms cells producing an enzyme converting glyoxal into glycolic acid, in a suitable culture medium comprising glyoxal as substrate, and optionally recovering glycolic acid from the culture medium.

By "**biochemical production**" in the present invention, it means a production involving biological material and preferably microorganisms cells, but which is not a fermentation process.

By "**glycolic acid derivatives**", it means compounds derived from glycolic acid used as an intermediate product, in particular compounds obtained from chemical polymerization, methylation or esterification of glycolic acid.

### ENZYME (GLYOXALASE)

By "**enzyme converting glyoxal into glycolic acid**", it means an enzyme that can convert glyoxal into glycolic acid according to the reaction: Glyoxal + H2O = Glycolic Acid + H⁺. The enzyme of the present invention converts glyoxal into glycolic acid, but also exhibits low activity to glycolic acid, for the said glycolic acid to be accumulated in the reaction system. In particular, the activity of the enzyme of the present invention to glycolic acid is preferably one-tenth of or less than, more preferably one-twentieth of or less than, and further preferably one-hundredth of or less than the activity thereof to glyoxal.

In particular, the enzyme of the present invention for converting glyoxal into glycolic acid is a glyoxalase, mainly characterized by the following physicochemical properties:
- acting on glyoxal to produce a corresponding glycolic acid; and
- exhibiting activity for glyoxal, but low or no activity for glycolic acid.

The enzyme of the present invention, glyoxalase, may further have the following physicochemical properties:
- molecular weight about 20 kDa in gel filtration analysis;
- optimum reaction temperature between 25 to 37°C; and
- optimum reaction at pH ranging from 5 to 8, preferably from 7 to 8.

The enzyme of the present invention may be selected in the following *in vitro* test:
- the enzyme is mixed with glyoxal in a buffer (ex: potassium phosphate buffer) at pH 7.5 for 0,10 to 60 minutes;
- after centrifugation and filtration, the samples were loaded onto the high-performance liquid chromatography (HPLC) column and glycolic acid production is spectroscopically monitored at 315nm.

In a particular embodiment, the glyoxalase is selected in the group consisting of the DJ-1 superfamily members of glyoxalases known in humans, worms, plants and bacteria. Preferably, the glyoxalase is selected from the group consisting of glyoxalases produced by *Escherichia coli* YajL, YhbO, and ElbB. In a preferred embodiment, the present invention uses an *Escherichia coli* glyoxalase III (GLY III); this enzyme is known to protect cells from thermal, oxidative, pH and UV stress (Abdallah et al., 2007). In a more preferred embodiment, the present invention uses the *E. coli* glyoxalase III encoded by YhbO gene (Gene ID : NP_417622.2), because it has a high affinity and activity on glyoxal (Km : 0,38mM ; kcat: 118,44 min⁻¹ ; kcat/Km : 3,11 × 10⁵ min⁻¹ M⁻¹, Lee et al., 2016).

So, in a preferred embodiment, the enzyme converting glyoxal substrate into glycolic acid is a glyoxalase having a catalytic triad Cys-His-Asp/Glu, in particular a glyoxalase III (GLYIII) comprising advantageously the conserved hallmark DJ-1_PfpA domain, preferably the glyoxalase III **(SEQ ID NO: 1)** encoded by YhbO gene from E. Coli **(SEQ ID NO:2)** or an amino acid sequence having at least 80% identity with SEQ ID NO:1.

In particular, the amino acid conserved DJ-1 Pfpl domain of YhbO comprises 168 amino acids as set forth in **SEQ ID NO: 3,** which corresponds to the nucleotide sequence represented by **SEQ ID NO :4.**

The table hereunder discloses the several sequences illustrated in the examples of the present invention, but the invention is not limited to said sequences.

**Table 1**

| **SEQ ID NO:** | **Sequence** | **Type of sequence** |
|---|---|---|
| 1 | MSKKIAVLIT DEFEDSEFTS PADEFRKAGH EVITIEKQAG KTVKGKKGEA SVTIDKSIDE VTPAEFDALL LPGGHSPDYL RGDNRFVTFT RDFVNSGKPV FAICHGPQLL ISADVIRGRK LTAVKPIIID VKNAGAEFYD QEVVVDKDQL VTSRTPDDLP AFNREALRLL GA | *E. coli* glyoxalase III protein encoded by YhbO gene |
| 2 | atgagtaaga aaattgccgt tttaatcact gatgaatttg aggattcaga atttacttca cccgcagacg agttccgtaa agccggacac gaagtgatta ccattgaaaa acaagcgggt aaaacggtga aaggcaaaaa aggagaagcc agcgtgacca tcgataaatc catcgatgaa gtgacgcctg cggagtttga tgccctgctg ctaccgggcg gccattcacc ggattatctg cgtggggaca accgttttgt cacctttacc cgtgattttg tgaatagtgg caaaccggtg tttgccatct gtcacggccc gcagttgctg atcagcgccg atgtgattcg ggggcgcaaa ctgaccgcag ttaaaccgat cattattgat gttaaaaatg cgggcgcgga attttacgat | Genomic DNA of *E.coli* YhBo gene |
| | caggaagtcg tggttgataa agatcagctg gttaccagcc ggacaccgga cgatctgcca gcgtttaacc gcgaggcgtt acgcctgctc ggtgcctga | |
| 3 | KKIAVLITDE FEDSEFTSPA DEFRKAGHEV ITIEKQAGKT VKGKKGEASV TIDKSIDEVT PAEFDALLLP GGHSPDYLRG DNRFVTFTRD FVNSGKPVFA ICHGPQLLIS ADVIRGRKLT AVKPIIIDVK NAGAEFYDQE VVVDKDQLVT SRTPDDLPAF NREALRLLG | conserved amino acid sequence of DJ-1 Pfpl domain of YhbO |
| 4 | aagaaaattg ccgttttaat cactgatgaa tttgaggatt cagaatttac ttcacccgca gacgagttcc gtaaagccgg acacgaagtg attaccattg aaaaacaagc gggtaaaacg gtgaaaggca aaaaaggaga agccagcgtg accatcgata aatccatcga tgaagtgacg cctgcggagt ttgatgccct gctgctaccg ggcggccatt caccggatta tctgcgtggg gacaaccgtt ttgtcacctt tacccgtgat tttgtgaata gtggcaaacc ggtgtttgcc atctgtcacg gcccgcagtt gctgatcagc gccgatgtga ttcgggggcg caaactgacc gcagttaaac cgatcattat tgatgttaaa aatgcgggcg cggaatttta cgatcaggaa gtcgtggttg ataaagatca gctggttacc agccggacac cggacgatct gccagcgttt aaccgcgagg cgttacgcct gctcggtgcc | conserved nucleotide sequence of DJ-1 Pfpl domain of YhbO |
| 5 | MKYLLPTAAA GLLLLAAQPA MA | pelB signal peptide amino acid sequence |
| 6 | atgaaatacc tgctgccgac cgctgctgct ggtctgctgc tcctcgctgc ccagccggcg atggcc | pelB nucleotide sequence |
| 7 | MKYLLPTAAA GLLLLAAQPA MAMSKKIAVL ITDEFEDSEF TSPADEFRKA GHEVITIEKQ AGKTVKGKKG EASVTIDKSI DEVTPAEFDA LLLPGGHSPD YLRGDNRFVT FTRDFVNSGK PVFAICHGPQ LLISADVIRG RKLTAVKPII IDVKNAGAEF YDQEVVVDKD QLVTSRTPDD LPAFNREALR LLGA | fusion protein pelB-YhbO |
| 8 | atgaaatacc tgctgccgac cgctgctgct ggtctgctgc tcctcgctgc ccagccggcg atggccatga gtaagaaaat tgccgtttta atcactgatg aatttgagga ttcagaattt acttcacccg cagacgagtt ccgtaaagcc ggacacgaag tgattaccat tgaaaaacaa gcgggtaaaa cggtgaaagg caaaaaagga gaagccagcg tgaccatcga taaatccatc gatgaagtga cgcctgcgga gtttgatgcc ctgctgctac cgggcggcca ttcaccggat tatctgcgtg gggacaaccg ttttgtcacc tttacccgtg attttgtgaa tagtggcaaa ccggtgtttg ccatctgtca cggcccgcag ttgctgatca gcgccgatgt gattcggggg cgcaaactga ccgcagttaa accgatcatt attgatgtta aaaatgcggg cgcggaattt tacgatcagg aagtcgtggt tgataaagat cagctggtta ccagccggac accggacgat ctgccagcgt ttaaccgcga ggcgttacgc ctgctcggtg cctga | fusion nucleotide sequence pelB-YhbO |

By '**80% identity',** it means 80, 81, 82, 83, 84, 85, 86, 87, 88, 89, 90, 91, 92, 93, 94, 95, 96, 97, 98, 99 or 100% identity.

The percentages of identity to which reference is made in the present invention are determined on the basis of a global alignment of sequences to be compared, that is to say, on an alignment of sequences taken in their entirety over their entire length, using any algorithm well-known to a person skilled in the art, for example the algorithm of Needleman and Wunsch 1970. This sequence comparison may be performed using any software well-known to a person skilled in the art, for example using the needle software by using the "Gap open" parameter equal to 10.0, the "Gap Extend" parameter equal to 0.5, and a "BLOSUM 62" matrix. The Needle software is for example available on the website ebi.ac.uk worldwide, under the name "Align".

When the glyoxalase according to the invention has an amino acid sequence that is not 100% identical to one of those described above but that has at least 80%, preferably at least 85%, at least 90%, at least 95%, at least 96%, at least 97%, at least 98%, at least 99% identity with one such reference sequence, it may have insertions, deletions or substitutions with regard to the reference sequence. When it is a matter of substitutions, the substitution is preferably made with an "equivalent" amino acid, *i.e.,* any amino acid whose structure is similar to that of the original amino acid and therefore unlikely to change the biological activity of the antibody. Such substitutions are also named 'conservative mutation' or 'conservative substitution'.

Examples of such substitutions are presented in **Table 2** below:

**Table 2. Substitutions with equivalent amino acids**

| **Original amino acid** | **Substitution(s)** |
|---|---|
| Ala (A) | Val, Gly, Pro |
| Arg (R) | Lys, His |
| Asn (N) | Gln |
| Asp (D) | Glu |
| Cys (C) | Ser |
| Gln (Q) | Asn |
| Glu (G) | Asp |
| Gly (G) | Ala |
| His (H) | Arg |
| Ile (I) | Leu |
| Leu (L) | Ile, Val, Met |
| Lys (K) | Arg |
| Met (M) | Leu |
| Phe (F) | Tyr |
| Pro (P) | Ala |
| Ser (S) | Thr, Cys |
| Thr (T) | Ser |
| Trp (W) | Tyr |
| Tyr (Y) | Phe, Trp |
| Val (V) | Leu, Ala |

In particular, the present invention relates to DNA encoding the aforementioned glyoxalase. Specifically, the present invention relates to DNA encoding the conserved hallmark DJ-1_PfpA domain of glyoxalase YhbO (**SEQ ID NO :3**), which has the nucleotide sequence represented by SEQ ID NO :4.

The present invention also relates to DNA encoding glyoxalase III *of E. coli* which has the nucleotide sequence represented by **SEQ ID NO: 2**. In a particular embodiment, the amino acid sequence of glyoxalase has 100% identity with the **SEQ ID NO:1**.

Furthermore, DNA encoding a protein comprising any amino acid sequence resulting from deletion, substitution, or addition of one or several amino acids in the amino acid sequence represented by **SEQ ID NO: 1** (amino acid sequence of glyoxalase III YhbO) or **SEQ ID NO :3** (amino acid sequence of Hallmark DJ1-1_PfpA domain of YhbO), is also included in the DNA of the present invention, as long as a protein having the protein encoded by the above DNA **SEQ ID NO :2** (nucleotide sequence of glyoxalase III YhbO ) or **SEQ ID NO :4** (nucleotide sequence of Hallmark DJ1-1_PfpA domain of YhbO) has activity of converting glyoxal into glycolic acid.

Methods of deletion, substitution, or addition of a specific amino acid(s) are well known form the skilled in the art, such as : PCR using synthetic DNA primers having nucleotide sequences comprising deletion of codons of specific amino acids, substitution with codons of other amino acids, or addition of codons of other amino acids; and a method of ligating an enzyme gene produced by known methods such as a chemical DNA synthesis method to a gene expression vector, and allowing it to express in a host such as *Escherichia coli* by genetic recombination.

The gene of an enzyme (e.g. glyoxalase) usable in the present invention can be obtained by well known methods, and in particular as following:
- purifying an enzyme protein from a microorganism that produces an enzyme converting glyoxal into glycolic acid, or from a culture broth thereof;
- using a peptide obtained by digesting the enzyme protein with protease, partial amino acid sequences are determined;
- using primers synthesized based on these partial amino acid sequences, PCR is carried out with genomic DNA as a template according to well known methods. By such PCR, a part of the enzyme gene is amplified, and the nucleotide sequence of the gene can be determined. Inverse PCR is also carried out using DNA primers synthesized from an N-terminal amino acid sequence and an amino acid sequence around the C-terminus, so as to determine a signal sequence, an N-terminal amino acid sequence, and a C-terminal amino acid sequence.

These different nucleic sequences encoding the enzyme (glyoxalase) are used in the present invention to be incorporated inside the vector (plasmid) used for transformation of microorganism cell (host) or for stably integrating the said nucleic sequence encoding the enzyme into the microorganism genome, using the well-known methods, as disclosed later in the description.

### BIOCATALYST (MODIFIED MICROORGANISM EXPRESSING THE SAID ENZYME)

By "**biocatalyst**", it means a catalyst made of biological material, in particular microorganisms cells having a catalysis activity.

Microorganisms used in the present invention are cultures of microorganisms or microorganisms cells capable of producing an enzyme converting glyoxal into glycolic acid.

By "**whole cell biocatalyst**" in the present invention, it means that the catalyst comprises or consists of whole cells of microorganisms expressing enzymes, as opposed to purified enzymes. Indeed, in the present invention, the whole cells are not lysed for extracting and purifying the expressed enzyme, but are used as such.

By "**microorganisms**" in the present invention, it encompasses bacteria, yeasts, fungi, actinomyctes, cells of animals or plants, but preferably bacteria sur as *Escherichia Coli,* in particular gram-negative bacteria for expression of the enzyme in the periplasmic space. By "**modified microorganism cells**", it means microorganisms cells that have been modified to express an enzyme converting glyoxal into glycolic acid, and preferably a glyoxalase. In the present invention, the microorganism cell is modified for expressing glyoxalase, meaning that the level and/or activity of said enzyme is increased in comparison to non-modified microorganism cell.

The expression 'modified microorganisms' encompasses 'transformed microorganisms' referring to a microorganism cell wherein exogenous nucleic acid has been incorporated.

The microorganism capable of producing the specific enzyme converting glyoxal into glycolic acid may be a wild strain, a mutant strain, or a transformant (recombinant) obtained (i) by incorporating the DNA of the enzyme (glyoxalase) into a vector (plasmid) and introducing it into the microorganism (host), or (ii) by stably integrating a DNA encoding the enzyme into the microorganism genome, using the well-known methods.

In a particular embodiment, the microorganisms, in particular *E. coli* strains such as the BL21(DE3) commercialized strain (Réf: C2527, NEB^{®}), illustrated later in the examples, are competent for transformation under the supplier protocol.

In another particular embodiment, *E. coli* strains such as MG1655 strain are rendred competent for transformation following the TSS protocol described by Chung and Miller (Chung et al., 1989). In fact, Chung and Miller developed a simple, one-step procedure for the preparation of competent *Escherichia coli* that uses a transformation and storage solution [TSS; 1 × TSS is LB broth containing 10% (wt/vol) polyethylene glycol, 5% (vol/vol) dimethyl sulfoxide, and 50 mM Mg2+ at pH 6.5]. Cells are mixed with an equal volume of ice-cold 2 × TSS and are immediately ready for use. Genetic transformation is equally simple: plasmid DNA is added and the cells are incubated for 5-60 min at 4 degrees C. A heat pulse is not necessary and the incubation time at 4 degrees C is not crucial, so there are no critical timing steps in the transformation procedure. Transformed bacteria are grown and selected by standard methods.

In a particular embodiment, the microorganisms cells are modified by transformation with an expression cassette being on a circular DNA (plasmid) or integrated into the microorganism genome.

In a particular embodiment, the expression cassette comprises a gene encoding a glyoxalase as defined above under the control of a constitutive promoter or an inductible promoter.

As '**constitutive promoter**', mention may be made of a proD promoter, promoter from Anderson constitutive promoter collection e.g J23100 to J23119 as referred to on the website: http://parts.igem.org/Promoters/Catalog/Anderson.

As '**inductible promoter**', mention may be made of an IPTG-inducible promoter, a photo-inducible promoter, a T5 promoter, a T7 promoter, a lac promoter (pLac), a lacT5 promoter, a lacT7 promoter, a tac promoter (pTac), a rhaBAD promoter, an araBAD promoter, a tet promoter, a penP promoter, a cspA promoter, or a promoter containing a tetO or lacO operator as an operator sequence.

In a particular embodiment, the inducible promoter is selected in the group consisting of a rhaBAD promoter, a lac promoter or a tac promoter.

In a particular embodiment, the enzyme converting glyoxal into glycolic acid is produced into the cytoplasmic space or alternatively into the periplasmic space of the microorganisms cells, preferably into the periplasmic space of the microorganisms cells.

In a particular embodiment, for example for expression of the glyoxalase enzyme into the periplasmic space of the microorganism, the microorganism is a gram-negative strain, in particular selected in the group consisting of Enterobacteriaceae, Alcaligenaceae, Vibrionaceae and Pseudomonadaceae, in particular Enterobacteriaceae, preferably Enterobacteriaceae belonging to the Salmonella, Yersinia or Escherichia genus, more preferably Escherichia genus, and even more preferably *Escherichia coli.*

In another particular embodiment, in particular for expression of the glyoxalase enzyme into the cytoplasmic space of the microorganism, other microorganisms may be used, such as gram prositive bacteria such as Actinomyces, Clostridium, Mycobacterium, Streptococci, Staphylococci, and Nocardia, fungi such as Saccharomyces and Candida species.

In the case of an enzyme exported across the cytoplasmic membrane of microorganism (also named produced in the periplasm of microorganism), a signal peptide is generally encoded in a portion corresponding to several tens of amino acids from the initiation codon of the gene (meaning upfront of the gene sequence). Such a signal peptide sequence is further cleaved so as to become a mature enzyme during a step in which an enzyme protein synthesized in the cells is exported across the cytoplasmic membrane of the cells.

In some cases, the native enzyme already comprises its own signal peptide, but generally man substitutes the original signal peptide sequence with another signal peptide sequence that is suitable for a host microorganism, so a chimeric gene can be constructed and used according to the method of the invention.

Several groups of proteins may be used as signal peptide. Mention may be made of soluble periplasmic proteins or those peripherally associated with the periplasmic side of the inner or outer membranes. In a particular and preferred embodiment, soluble periplasmic proteins are used. In another particular and preferred embodiment, a protein associated with the periplasmic side of the inner or outer membranes, such as NIpA, that allows anchorage to the membrane, is used.

As '**signal peptide**', mention may be made of dsbA, EOX, lamB, MgIB, MmAp, ompC, ompT, sufl, SfmC, STII, tolB, torA, torT, glll, malE, ompA, pelB, phoA, and nlpA, preferentially pelB.

In a particular embodiment, pelB signal peptide amino acid sequence **(SEQ ID NO: 5)** encoded by signal peptide nucleotide sequence (**SEQ ID NO: 6**) are used in the present invention.

So, in the case of an enzyme to be produced in the cytoplasm of the cells of a microorganism used as a host, man uses an enzyme gene without adding a nucleotide sequence encoding a signal peptide or from which a signal peptide sequence has been removed.

And in the case of an enzyme to be produced in the periplasm of the cells of a microorganism used as a host, man uses an enzyme gene wherein a nucleotide sequence encoding a signal peptide replaces the original signal peptide or has been added upfront the enzyme gene.

So, in a particular embodiment, the present invention also provides expression cassette comprising an enzyme gene (YhbO gene, **SEQ ID NO:2**), without any signal peptide sequence for producing enzyme in the cytoplasm of a microorganism used as a host.

And in other particular embodiment, the present invention also provides expression cassette comprising an enzyme gene (YhbO gene, **SEQ ID NO:2**) and a signal peptide sequence (pelB, **SEQ ID NO: 5**) upfront of the enzyme gene, forming a fusion nucleotide sequence pelB-YhbO (**SEQ ID NO: 8**) for producing enzyme in the periplasm of the cells.

In this case, a fusion protein 'signal peptide-enzyme' (pelB-YhbO) (**SEQ ID NO: 7**) is produced by the host microorganism but the signal peptide is further cleaved so that the native glyoxalase III protein produced in the periplasm is soluble. In a particular and preferred embodiment, the glyoxalase is expressed in the periplasmic space of the microorganism cell. The translocation to the periplasm of the enzyme offers several advantages compared to cytosolic (cytoplasmic) production such as avoidance of protease attack and better chance of correct protein folding in an oxydizing subcellular environment. Numerous periplasmic proteins have been shown to be involved in the folding and prevention of aggregation of proteins. These includes Dsb (Disulfide bond forming) proteins that form and isomerize disulfide bonds, PPlases that catalyze the trans→ cis isomerization of peptidyl-prolyl bonds, general chaperones such as FpkA (Ehrmann, 2007). The formation of disulfide bounds can result in a tightly folded structure that is resistant to proteases.

In this particular embodiment, the enzyme is fused to a signal peptide to adress the enzyme into the periplasmic space. Most proteins can be successfully translocated across the cytoplasmic membrane to the periplasm. When conditions are optimized for periplasmic accumulation, a huge percentage of the total cell protein can be exported to the periplasm, percentages in the 20% to 40% range can be attained (Ehrmann, 2007).

So, in a particular embodiment, the expression cassette also comprises a signal peptide for addressing the glyoxalase into the periplasmic space, in particular selected in the group consisting of dsbA, EOX, lamB, MgIB, MmAp, ompC, ompT, sufl, SfmC, STII, tolB, torA, torT, glll, malE, ompA, pelB, phoA, nlpA, preferably pelB.

So, the present invention also concerns a whole cell biocatalyst comprising or constituting of modified microorganisms cells producing a enzyme able to convert glyoxal into glycolic acid, as defined above.

The present invention also relates to a method of producing a whole cell biocatalyst as defined above, comprising the steps of:
a) Transforming the microorganisms cells by a plasmid comprising an expression cassette comprising a gene encoding an enzyme converting glyoxal into glycolic acid under the control of a constitutive or inducible promoter, and comprising optionally a signal peptide for addressing the said enzyme into the periplasmic space; or alternatively a') engineering the microorganisms cells by integrating the said expression cassette into their genome;
b) Cultivating the modified cells in a suitable culture medium for the expression of the said enzyme;
c) Separating the cells from the supernatant by centrifugation; and
d) Optionally drying the cells and storing them at 4°C or -20°C or -80°C.

In a particular and preferred embodiment, the microorganisms cells (host) are *E. coli* strains, preferably E. coli BL21(DE3) strains or MG1655 strains, and the enzyme is glyoxalase III (GLY III) encoded by YhbO gene of *E. Coli* (in particular **SEQ ID NO: 1** is the amino acid sequence of GLY III of E. Coli and **SEQ ID NO: 2** is the nucleotide sequence of YhbO gene).

Another object of the invention is an expression cassette comprising a nucleotidic sequence encoded the enzyme as defined above under the control of a promoter, preferably an inducible promoter (eg IPTG) as disclosed above.

In a particular embodiment, in particular for expressing the glyoxalase in the periplasm of the modified microorganism cells, the expression cassette comprising a nucleotidic sequence encoding the glyoxalase as defined above and also a signal peptide sequence encoding a signal peptide as defined above for adressing the said glyoxalase into the periplasmic space of a microorganism cell.

In a particular and preferred embodiment, the signal peptide is pelB (**SEQ ID NO :5**) encoded by the signal peptide pelB gene (**SEQ ID NO: 6**).

Another object of the invention concerns a nucleotide sequence encoding the polypeptide (enzyme) or fusion polypeptide (with signal peptide) as defined above.

In a particular embodiment, the expression cassette comprises a fusion nucleotide sequence pelB-YhbO as set forth in **SEQ ID NO :8** comprising pelB signal peptide and YhbO gene, for producing a fusion polypeptide (or fusion protein pelB-YhbO) as set forth in **SEQ ID NO: 7.**

The invention also relates to a vector comprising a nucleotide sequence as defined above.

### IMMOBILIZATION OF THE BIOCATALYST (optional)

The modified microorganisms cells may be free in the culture medium or advantageously immobilized. Immobilization can be performed by methods well known to those skilled in the art (eg, cross-linking method, physical adsorption method, entrapment method, etc.). In a particular embodiment, microorganisms cells are immobilized on agar or carrageenan, or alternatively the microorganisms cells are crosslinked, in particular with glutaraldehyde (GA) and/or polyethylenimine (PEI), or are both immobilized and crosslinked.

In a particular embodiment, the biocatalyst is immobilized on agar or carrageenaan or crosslinked with glutaraldehyde and/or polyethylenimine.

The immobilization of the whole cell biocatalyst permits to easily recycle the said biocatalyst for a further use in another reaction.

In a particular embodiment, the microorganisms cells are encapsulated in carrageenan beads. Such embodiment is particularly advantageous for bioproduction in the periplasm space of the microorganisms.

In a particular embodiment, the microorganisms cells are immobilized with glutaraldehyde and/or polyethylenimine.

The immobilization of the modified microorganisms cells is advantageous for recycling the modified microorganisms cells for another further reaction.

### CONDITIONS OF CULTURE AND CONVERSION REACTION

By "**suitable culture medium**", it means a medium suitable for proliferation of the microorganisms. The medium is generally a liquid, gelled or solid medium which comprises carbon sources including sugars, alcohols, nitrogen sources and others compounds needed for the proliferation of the said microorganisms and the reaction of conversion of the glyoxal also present in the medium as substrate, into glycolic acid. Examples of suitable medium (TB medium) will be disclosed later in the description.

The reaction conditions are different depending on the enzyme used, the microorganism used, and the processed product thereof.

In a particular embodiment, for producing glyoxalase III in a culture of *E. coli,* the temperature is between 25°C and 37°C, and preferably 25°C ; the pH is between pH 5 and 10, and preferably between pH 7 and 8. The reaction is preferably carried out under conditions consisting of shaking and agitation (100rpm to 600rpm, preferably around 200rpm). And the time for reaction is generally comprised between one and fifty hours, preferably between one and three hours.

in a particular embodiment, the ratio cell dcw*: substrate is between 1 and 30%, preferably between 1% to 10%, more preferably between 1% to 5% and even more preferably 1% in a variable reaction volume.
*dcw= dry cell weight.

In a particular embodiment considering a glyoxal solution a 40%, the maximal concentration of glyoxal used is 8,7 M.

The substrate used in the reaction is glyoxal.

Glyoxal is the smallest dialdehyde. Glyoxal is soluble in water, it forms an equilibrium between mono- and dihydrate forms. It can also form dimers and oligomers by condensation (reversible). Glyoxal can be produced by gas phase oxidation of ethylene glycol by air in the presence of a copper or iron catalyst. This process is used by BASF (Ludwigsafen, Germany) with a reported production volume of 60 kt/year (BASF, 2016). Another process is based on the liquid phase oxidation of acetaldehyde by nitric acid, WeyChem thus produces glyoxal, used for the production of glyoxylic acid. Acetaldehyde is produced from the oxidation of ethylene or ethanol. The production of ethylene glycol and biobased ethanol has created growing interest, glyoxal can now be synthesized from renewable resources.

In a particular embodiment, the glyoxal is directly added in the culture medium. For example, the glyoxal is an aqueous solution of glyoxal (40%).

The reaction comprises combining the set of reaction components under suitable reaction conditions whereby glycolic acid is produced in aqueous solution or organic solvant (i.e methanol).

Production of glycolic acid in methanol present some advantages:
- Reduction of water use ;
- Methanol evaporation is less energy-intensive than that of water
- Production of glycolate derivatives such as methylglycolate: the esterification reaction would be potentially limited by the excessive presence of water.

The reaction may be managed in a batch or fed-batch mode, repeated or not.

The '**batch mode**' is where no extra feeding is used from beginning to end of the process.

The '**fed-batch mode**' is where feeding with substrate (glyoxal) and supplements can extend the duration of culture for higher cell densities to produce the glycolic acid.

The '**repeated Fed-batch**' is where harvesting all but a small residue of a completed (fed) batch and leaving the remaining cells available is to use as an inoculum for the next batch. Mention may be made also of continuous mode for bioconversion, wherein the incoming feed rate matches the rate of removal of harvest, with cell retention.

### PRODUCT

The glycolic acid produced according to the method of the invention may be recovered.

By "**recovering the glycolic acid from the medium**", it means a step of separation, extraction, filtration and/or purification of the glycolic acid from the medium. Generally, this step comprises centrifugation, microfiltration, cation and anion exchanges, and evaporation.

In a particular embodiment, the separation solid/liquid is carried out by centrifugation between 4000 to 5000 rpm, in particular at 4500 rpm, for a time ranging from 5 to 15mn, in particular 10 min and the microfiltration is made with filter having porosity ranging from 0,1 to 0,2µm, in particular 0,2 µm.

In a particular embodiment, the extraction of glycolic acid is carried out by anion exchange chromatography.

In a particular embodiment, the extraction of glycolic acid from the supernatant was carried out by anion exchange chromatography with a ion-exchange column such as Dowex resin columns (ex: Dowex 1x8 resin form OH).

As an illustrative example, first, sample was added to the ion-exchange column at a speed of 1 to 5 mL/min, in particular 2,5 mL/min using a peristaltic pump. Then, the ion-exchange resin column was cleaned by deionized water. A sequential elution process with NaOH at different concentrations (60, 120, 200 mM) was carried out. For subsequent uses, the stored ion-exchange resins were cleaned by 1 M NaOH.

The fractions of interest are then eluted on a cation exchange resin (ex: DOWEX 50WX8 H form) in order to convert sodium glycolate to glycolic acid.

In a particular embodiment, the evaporation is performed by an evaporator (Buchi R-215) at around 40° C at around 40 mbar, then completed by freeze-drying (Epsilon 2-4 LSC).

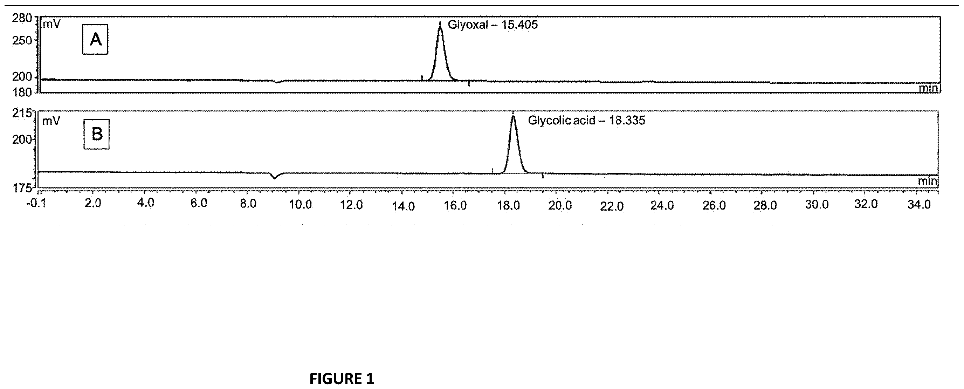

Quantification of glyoxal and glycolic acid can be carried out by high performance liquid chromatography (HPLC). In a particular embodiment, the concentrations of glyoxal (substrate) and glycolic acid (product) were measured by UHPLC (Dionex UltimateTM 3000) equipped with a Phenomenex ROA- Organic Acid H+ (8%) column (300x7.8mm) column and pre-column (50x7.8mm) and 5 mM H₂SO₄ was used as a mobile phase at 0,5 mL/min for 35 minutes. Detection is performed by a refractometer (Shodex RI-101) and a UV detector (Dionex UltiMate 3000 Diode Array Detectors 3000(RS). The samples were filtered at 0.2 µm. Under the present conditions, and as illustrated in **Figure 1**, glyoxal is eluted at 15 minutes, and glycolic acid is eluted at 18 minutes.

The extraction of glycolic acid may further be followed by analysis of the quality and properties of the glycolic acid as produced.

In particular, NMR (Nuclear Magnetic Resonance spectroscopy) technique permits the observation of local magnetic fields around atomic nuclei and such analysis is used to obtain high resolution information of the product.

In particular, the NMR analyses applied on the glycolic acid produced by the method of the invention show that the majority product is glycolic acid. Only 0,05% impurity was found in proton NMR.

In another hand, IR (Infrared spectroscopy or vibrational spectroscopy) technique is used to study and identify compounds by the measurement of the interaction of infrared radiation with matter by absorption, emission or reflection.

In the present invention, the IR analyses applied on the glycolic acid produced by the method of the invention show a correlation of 95% with a commercial standard of glycolic acid.

Mass spectrometry (MS) was also used to confirmed the molecular weight and the molecular formula of glycolic acid

So, in a particular embodiment, the method of the invention further comprises a step of recovering glycolic acid from the culture medium, and optionally a step of analysing the glycolic acid as produced, preferably by NMR, MS and/or IR analyses.

And in a particular embodiment, the glycolic acid produced according to the invention is characterized by the following properties:
- it comprises less than 30% impurity, preferably less than 0,05% impurity on proton NMR;
- its structure and quality show a correlation of more than 90%, preferably of 95% in IR with a commercial standard of glycolic acid;
- a molecular weight of 75,0082 (+/- 1mDa) in mass spectrometry in negative mode for the specie [M-H] and an expected formula C2H3O3.

The glycolic acid as produced may be used as such, or may be used as an intermediate product in a further chemical reaction, such as esterification or polymerization.

So, the present invention also concerns a method of producing a glycolic acid derivative or a product obtained by a reaction using glycolic acid as substrate, comprising at least a step of the method for the biochemical production of glycolic acid as defined above.

The method may also further comprises a step of recycling the modified microorganisms cells producing an enzyme converting glyoxal into glycolic acid (biocatalysts), at the end of the bioconversion reaction.

Generally, the modified microorganisms cells can be recycled 2 to 4 times.

The recycling generally comprises a step of washing the cells with PBS and harvesting them for further use.

### BRIEF DESCRIPTION OF THE FIGURES

**Figure 1****:** HPLC-RI chromatograms of standard solution of (A) Glyoxal 10 g/L; (B) Glycolic acid 10 g/L.
**Figure 2****:** Production of glycolic acid in variable volume (2 to 2000 mL).
**Figure 3****:** Kinetics of glycolic acid production according to the volume of production.
**Figure 4****:** Kinetics of glycolic acid production and glyoxal consumption.
**Figure 5****:** Glycolate production in 700 mL reactor with continuous substrate supply.
**Figure 6****:** Glycolate production in 2000 mL reactor with substrate feed.
**Figure 7****:** Production of glycolate with free resting cells and crossed-linked resting cells
**Figure 8****:** Bioconversion % after 2 hours in batch mode comprising both free and cross-linked cell system.
**Figure 9****:** Glycolate production by carrageenan immobilized cells.
**Figure 10****:** Bioconversion % after 2 hours in batch mode comprising both free and treated cells using glurataldehyde (GA) and/or polyethylenimine (PEl).

The present invention will be now illustrated by the following and non-limitated examples.

### EXAMPLES

### Example 1: Material and Methods

### 1.1 Materials

### 1.1.1 Plasmids for expression of glyoxalase into the periplasmic space or cytoplasmic space

Different plasmids were prepared:
1) **pET28b-yhbO**, a plasmid designed for cytoplasmic production of YhbO enzyme, IPTG inductible, is prepared as following: pET28a plasmid (ori pBR322, kanamycin, T7 promoter) from Novagen ^{®} (Ref 69864) is used for the expression of the *yhbO* gene (SEQ ID NO: 2) from *Escherichia coli* encoding a deglycase (glyoxalase) into the cytoplasm of the cell. The yhbO gene is extracted with the genomic DNA purification Kit (ThermoFisher Ref: K0721) from Escherichia coli MG1655 (ATCC 700926), then amplified by PCR (Polymerase Chain Reaction) using the appropriate condutions and primers, then inserted into linearized plasmid by homologous recombination. The primers used for gene amplification are of generally known type. A person skilled in the art knows how to choose them and use them in the context of PCR techniques.
2) **pET22b-yhbO**, a plasmid designed for periplasmic production of YhbO enzyme, IPTG inductible, is prepared as following: pET22b plasmid (*ori* pBR322, ampicillin, T7 promoter, pelB) from Novagen ^{®} (Ref 69744) is carrying the signal sequence pelB at the N-terminal and a His-tag sequence at the C-terminal. This plasmid is used for the expression of the *yhbO* gene from *Escherichia coli* encoding a deglycase (glyoxalase). The yhbO gene is extracted with the genomic DNA purification Kit (ThermoFisher Ref: K0721) from Escherichia coli MG1655 (ATCC 700926), then amplified by PCR (Polymerase Chain Reaction) using the appropriate conditions and primers (Taq *2X Master Mix (M0270) (NEB* ^{®}*)*, then inserted into linearized plasmid by homologous recombination. The primers used for gene amplification are of generally known type. A person skilled in the art knows how to choose them and use them in the context of PCR techniques.
3) pET28b is a similar plasmid as pET28a-yhbO but without yhbO gene, and is used as a negativ control.

### 1.1.2 Construction of strains (biocatalysts)

The different types of biocatalysts (transformed E. coli strains) were produced and used in the following examples.

Four *E.coli* BL21 (DE3) (Réf: C2527, NEB^{®}) strains were prepared, carrying respectively the above plasmids :
1) **pET28b-yhbO,** a plasmid designed for cytoplasmic production of YhbO enzyme, IPTG inducible ;
2) **pET22b-yhbO,** a plasmid designed for periplasmic production of YhbO enzyme, IPTG inducible ; or
3) **pET28b** empty as a negativ control.

Competent commercial cells such as BL21(DE3) (Réf: C2527, NEB^{®}) were transformed according to the supplier's protocol. The strains used are given in **Table 3** hereunder.

**Table 3 : E. coli strains used**

| **Strain** | ***Genotype*** | ***Plasmid*** | ***Resistance*** | ***Expression of the enzyme*** |
|---|---|---|---|---|
| BL21-YhbO | *E.coli str. BF- ompT gal dcm lon hsdSB(rB-mB-) λ(DE3 [lacI lacUVS-T7p07 ind1 sam7 nin5]) pelB-yhbO* | *pET22b-yhbO* | *Amp* | *Periplasmic under IPTG inducible promoter* |
| BL21-YhbO | *E.coli str. BF- ompT gal dcm lon hsdSB(rB-mB-) λ(DE3 [lacI lacUVS-T7p07 ind1 sam7 nin5]) yhbO* | *pET28b-yhbO* | *Kan* | *Cytoplasmi c under IPTG inducible promoter* |
| BL21 | *E.coli str. BF- ompT gal dcm lon hsdSB(rB-mB-) λ(DE3 [lacI lacUV5-T7p07 ind1 sam7 nin5]) yhbO* | *pET28b* | *Kan* | *Cytoplasmi c under IPTG inducible promoter (control)* |

Glyoxalase-producer BL21(DE3) strains ('biocatalysts') were obtained through a two-step enrichment cultivation procedure on TB medium of following composition (per liter): 24 g yeast extract, 20 g tryptone, 17 mmol KH2PO4, 72 mmol K2HPO4, 4 g glycerol, 10 mg FeSO4, 2 mmol MgSO4, 1 mmol CaCl2. The strain after being grown on solid medium (10 g/L tryptone, 5 g/L yeast extract, 10 g/L NaCl, 15 g/L agar) overnight at 37°C, were first pre-cultivated in 10 ml TB medium for 4h at 37°C, 200 rpm, then inoculated in 500 mL culture in 2L Erlenmeyer flask and shaken at 37°C and 200 rpm. Expression of glyoxalase was induced by 0,1 mM isopropyl-B-D-1-thiogalactopyranoside (IPTG) at mid-exponential phase (0,4 <DO600 nm <0,6) and shaken at 20°C and 200 rpm overnight, approximately for 16h. The cell pellet was harvested by centrifugation 4500 rpm for 10 minutes. The standard established relationship between dry cell mass per mL and optical density at 600nm following a standard protocol is as follows: Dry weight cell (dwc) (mg/ml) = 0.6897 * OD ₆₀₀ₙₘ

The Dowex resin and chemicals used for production, purification and analytical standards, such as NaOH, HCl, H2SO4, NaCl, KCI, glyoxal, glycolic acid, were obtained from Sigma Co. Other solutions of glutaraldehyde (GA), polyethylenimine (PEI), and carrageenan were commercially available (Sigma-Aldrich ^{®}).

### 1.2 Whole cell catalysis reaction

Whole cells as prepared above (E. coli strains transformed with plasmids, and constituting the 'biocatalysts') are centrifuged at 4500 rpm for 10 minutes and resuspended in water. As disclosed in some other following examples, the whole cells as prepared above can also be immobilized using glutaraldehyde and/or polyethylenimine for facilitating the further recycling of the biocatalysts.

The cells carrying YhbO gene (used as 'biocatalysts') are introduced into the reaction mixture. This includes a buffer to stabilize the pH and the glyoxal substrate. The composition is deliberately as simple as possible in order to ensure optimum enzymatic activity and minimized production cost.

The glycolic acid production reactions are placed at 25° C with stirring (200 rpm). The composition of the reaction mixture is: 100 mM HEPES buffer pH 7.5, 100 mM glyoxal. The production is carried out in volumes of 2 mL, 20 mL, 200 mL and 2000 mL in a 2mL Eppendorf tube, 50 mL, 500 mL and 5000 mL flask, respectively. Negative controls without substrate (glyoxal) are carried out as well as negative controls (cells transformed with empty plasmid). Samples are collected by taking 300 µl of the reaction medium, centrifuged at 20,000 g for 2 minutes and then filtered (0.2 µm).

Fed-batch catalysis technique was further tested for the bioconversion of glyoxal to glycolic acid. A bio-production of glycolic acid was conducted in fed-batch in 700 mL reactor (Solaris ^{®}) with a catalytic broth containing phosphate buffer at an initial concentration of 200 mM, at pH 7.5 and 30° C., stirring by a single blade of the Rushton type with 6 blades at 1000 rpm, an air flow of 150mL /min. The initial volume of reaction mixture was set at 300 mL in order to have enough volume available for feeding and pH regulation. Glyoxal (6,2 M) was added continuously by peristaltic pump at 0,59 mL/min. The pH was adjusted to 7,5 by 3 M NaOH.

Another bio-production of glycolic acid was conducted in 2000 mL reactor (Sartorius ^{®}).

The reaction was conducted in an initial concentration of 200 mM of phosphate buffer at pH 7.5 and 25° C., stirring by two 6-blade Rushton type blades at 600 rpm, an air flow of 100 mL /min. The initial volume of reaction mixture was set at 500 mL. Glyoxal (8,7M) was added continuously by peristaltic pump. Flow adjustment was done according to the quantity of glycolate produced in 1 hour. The pH was adjusted to 7,5 by 8,3 M NaOH.

After one batch cycle, the catalysis broth and *E.coli* cells (biocatalysts) were separated by centrifugation at 10,000 g for 15 minutes.

### 1.3 Analytical methods

The concentrations of glyoxal (substrate) and glycolic acid (product) were measured by UHPLC (Dionex UltimateTM 3000) equipped with a Phenomenex ROA- Organic Acid H+ (8%) column (300x7.8mm) column and pre-column (50x7.8mm) and 5 mM H₂SO₄ was used as a mobile phase at 0,5 mL/min for 35 minutes. Detection is performed by a refractometer (Shodex RI-101) and a UV detector (Dionex UltiMate 3000 Diode Array Detectors 3000(RS). The samples were filtered at 0.2 µm.

The structural confirmation analyzes were carried out by the Toulouse Institute of Chemistry (ICT) by nuclear magnetic resonance (NMR), infrared (IR) and mass spectrometry (MS).

### 1.4 Ion exchange chromatography

The extraction of glycolic acid was carried out by anion exchange chromatography with a Dowex 1x8 resin form OH-, with a capacity of 1.2 mEq / mL. 8 mL or 120 mL columns connected to a peristaltic pump were used to contain the resin. First, sample was added to the ion-exchange column at a speed of 2,5 mL/min using a peristaltic pump. Then, the ion-exchange resin column was cleaned by deionized water.

A sequential elution process with NaOH at different concentrations (60, 120, 200 mM) was carried out. For subsequent uses, the stored ion-exchange resins were cleaned by 1 M NaOH. The fractions of interest are then eluted on a cation exchange resin (DOWEX 50WX8 H form) in order to convert sodium glycolate to glycolic acid.

### 1.5 Drying and evaporation of water

Evaporation was first performed using a rotary evaporator (Buchi R-215) at 40° C. at 40 mbar, then completed by freeze-drying (Epsilon 2-4 LSC).

### 1.6 Full mass spectrometry analyses of the product (glycolic acid)

The device used is a "XevoG2QTof" mass spectrometer from the company Waters, equipped with an electrospray ionization (ESI) source and a time-of-flight (Tof) analyzer, allowing the determination of the exact mass to the 4th decimal and the proposal of raw formulas.

The exact mass pf the Species were detected in negative ion mode [M-H]- i.e. 75.0082 at less than 1mDa.

### Example 2: Results

The reaction scheme for the production of glycolic acid from glyoxal is as follows:

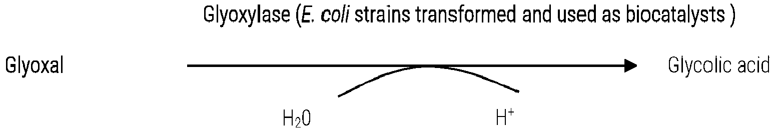

### 2.1 Production of glycolic acid from glyoxal in volumes of 2mL to 2000mL using a biocatalyst in periplasmic space

The production of glycolic acid from glyoxal is carried out in volumes of 2 mL, 20 mL, 200 mL and 2000 mL. The reacted mixture consists of the following elements:
- Biocatalyst: 2 DO/ml of BL21(DE3) pET22b-YhbO cells
- Substrate: glyoxal (100mM)
- Buffered solution: HEPES (100 mM)

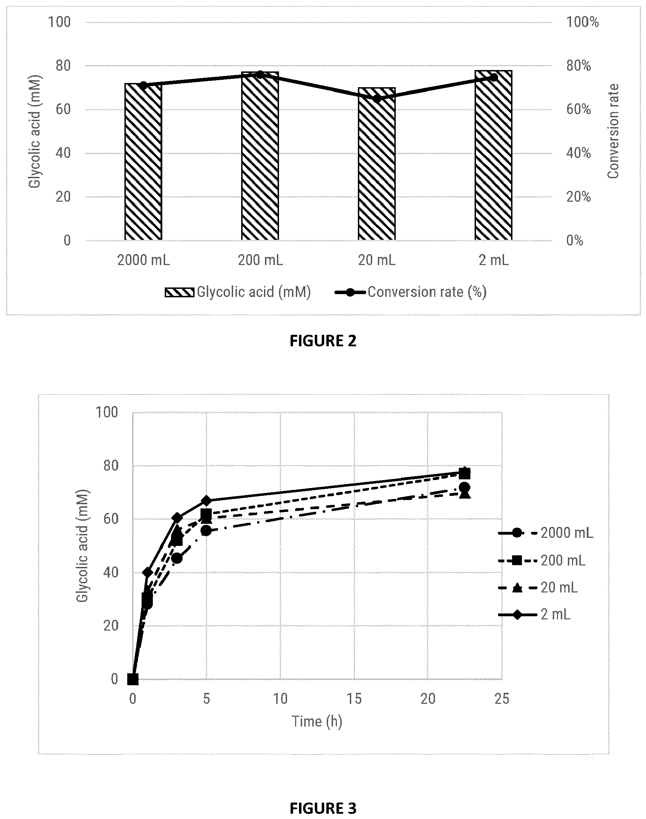

In 20 h, 74 mM of glycolic acid are produced, with a conversion rate of 75%. UPLC analysis indicates that the glyoxal is transformed into glycolic acid without coproduct formation whatever the scale of production **(****Figure 2****),** moreover glycolic acid production kinetic profile remains identical for the four volumes tested (**Figure 3**). Increasing the volume by a factor of 1000 is therefore possible without loss of efficiency.

Production with a higher quantity of catalyst (16 DO/ml instead of 2 DO/ml) are carried out under the following conditions:
- Biocatalyst: 16 DO/ml of BL21(DE3) pET22b-YhbO cells
- Substrate: glyoxal (100mM)
- Buffered solution: HEPES (100 mM)
- Volume: 2mL

We observe a production of 100 mM or 7.6 g/L of glycolic acid in 3 hours with a yield of 100%.

The whole cell biocatalyst comprising BL21-YhbO cells is therefore an effective tool for the production of glycolic acid from glyoxal. We demonstrated a stable glycolic acid production of 5.7 g/L with a conversion rate of 75% for volumes ranging from 2 ml to 2000 ml. By optimizing the quantities of biocatalyst used, a production of 7.6 g/L of glycolic acid from 5.8 g/L of glyoxal with a yield of 100% in 3 hours has been achieved.

### 2.2 Production of glycolic acid from glyoxal in Fed batch catalysis

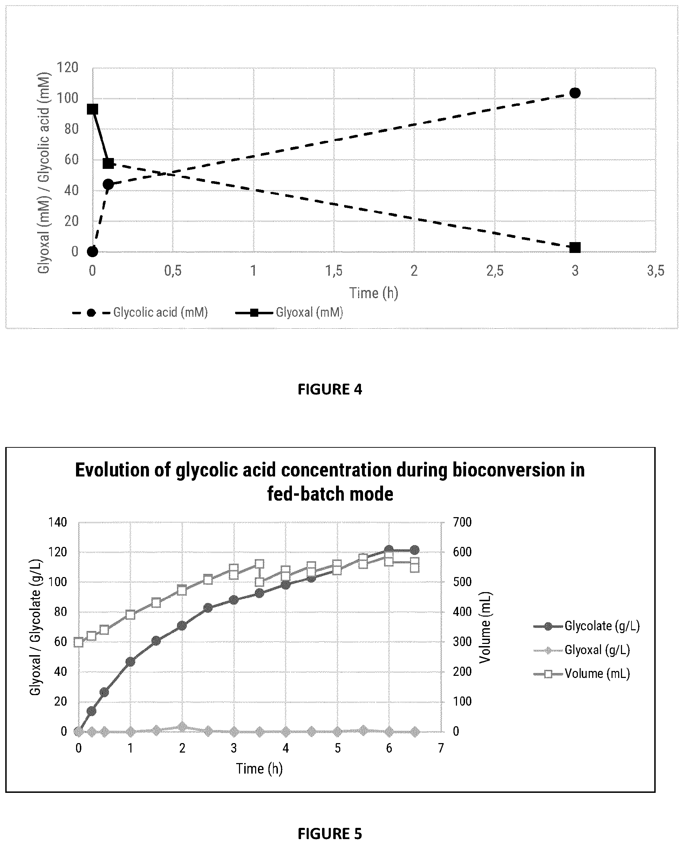

Fed-batch catalysis technique was further tested for the bioconversion of glyoxal to glycolic acid. A first fed -batch reaction was carried out in a 700 mL capacity reactor (**Figure 5**). Parameters are described in part 1.2. A fed -batch catalysis with limitation of substrate accumulation was implemented, thus glyoxal was immediately consumed.

A production of 120 g/L of glycolate was observed in 6,5 hours with a yield of 99% without residual glyoxal (**Table 4**). The reaction could not be continued beyond this point, the maximum volume of the reactor having been reached. The pH regulation with 3M NaOH constitutes nearly 60% of the volume addition during the reaction.

**Table 4. Performance of the biocatalyst for the production of glycolate in a 700 mL capacity reactor**

| | |
|---|---|
| Final volume | 547 mL |
| Glycolate concentration in the reactor after 7 hours | 121 g/L |
| Quantity of glycolate in the reactor after 7 hours | 66 G |
| Mass yield | 1.29 g/g |
| Molar yield | 0.99 mol/mol |
| Product/catalyst ratio | 3.29 gGlycolate / gCells |
| Catalyst/product ratio | 30% gCells / gGlycolate |

The production of glycolate in the reactor is promising with a productivity of 23 g/L/h, and several improvement points would even optimize the performances:
- NaOH concentration can be increased to limit the increase in volume;
- A larger capacity reactor can be used to allow for higher volume production and finer control of base, acid, and feed rates;
- The temperature can be lowered to reduce the costs of the process; and
- The quantity of cells can be optimized to reduce costs.

As a consequence, the following fed-batch bioconversion was carried out with said improvement points, ie an increased NaOH concentration at 8.3 M, a reactor capacity 2.5 times higher, a temperature lowered to 25°C and a final catalyst concentration reduced by 19%.

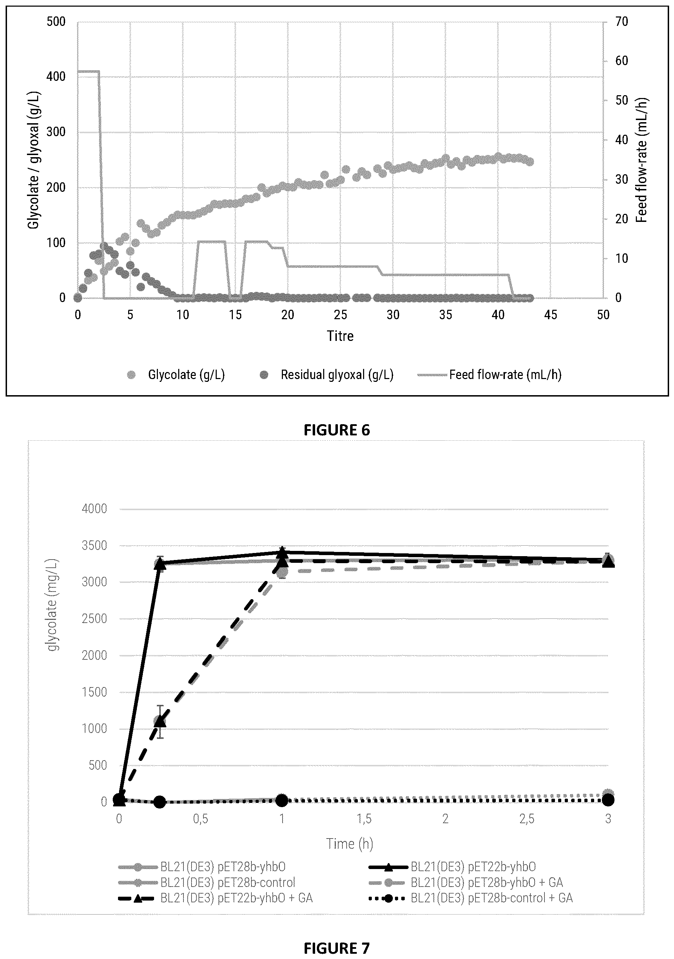

So, a second fed -batch reaction was carried out in a ROPA type reactor with a capacity of 2000 mL **(****Figure 6****)**.

The main performance data are described in **Table 5**. Finally, 247 g/L of glycolic acid were obtained at a yield of 98% (equivalent to 1.29 g glycolate / g glyoxal) and a volume productivity of 5.7 g/l/h in 43h that presently is the greatest level of glycolic acid production reported. A quantity of 334 g of glycolate was produced from 259 g of glyoxal and 44.6 g of biocatalyst. No residual glyoxal was observed at the end of the reaction.

The specific activity of the biocatalyst is 0.37 g _{glyoxal}/ g _{biomass}/h at the start of the reaction against 0.28 g _{glyoxal}/ g _{biomass}/h at the end of the reaction. This may be caused by the accumulation of glyoxal at the start of the experiment which may have damaged the cells or the clogging of the fixed parts of the reactor by a mass of cells making part of the biomass unavailable for the reaction despite the strong agitation.

**Table 5. Performance of the biocatalyst in a 2L reactor with glyoxal feed and pH regulation at 7.5 over 43h of reaction**

| | |
|---|---|
| Final volume | 1086 mL |
| Glycolate concentration in the reactor at 43h | 247 g/L |
| Quantity of glycolate in the reactor at 43h | 268 g |
| Mass yield | 1.29 g/g |
| Molar yield | 0.98 mol/mol |
| Product/catalyst ratio | 7.48 gGlycolate / gCells |
| Catalyst/product ratio | 13% gCells / gGlycolate |

### 2.3 Structural confirmation by nuclear magnetic resonance (NMR), infrared (IR) and mass spectrometry (MS)

Purified glycolic acid obtained by ion exchange chromatography was used for structural analyzes. NMR (Proton, Carbon, 2D HSBC and 2D HSQC), IR and MS analyses were performed by Toulouse Institute of Chemistry.
- NMR analyses show that the majority product is glycolic acid. Only 0,05% impurity was found in proton NMR.
- IR analyses show a correlation of 95% with a commercial standard of glycolic acid. Difference in crystalline form may explain this difference.
- Full mass spectrometry (MS) analyses show that the raw formula obtained by mass spectrometry analyses of the glycolic acid produced by the process of the invention corresponds to the expected raw formula, that is C2H3O3, meaning a perfect correlation between the commercial standard and the glycolic acid as produced.

The production of glycolic acid at 247 g/L at a yield of 99% with a volume productivity of 5.7 g/l/h exceeds the performance of all published fermentation processes. The process temperature was also successfully reduced from 30°C to 25°C; making the operating conditions competitive from an energy point of view. After anion exchange chromatography, purified product was recovered with an HPLC purity >99%. Three structural analyzes confirmed that high purity glycolic acid was obtained, with only 0,05% impurities found in NMR. The purified glycolic acid is a white crystalline powder, without traces of formaldehyde, suitable in particular for cosmetic grade.

The following examples illustrate the production of glycolic acid from glyoxal, using biocatalysts (whole cells) producing glyoxalase into cytoplasmic vs periplasmic of the said biocatalysts, with or without immobilization.

### 2.4 - Effect of Periplasmic vs Cytoplasmic biocatalyst and effect of biocatalyst immobilization on the bioconversion reaction

Three types of catalysts were produced as disclosed in example 1.2, all were *E.coli* BL21(DE3) strains carrying different plasmids 1) pET28b-yhbO, a plasmid designed for cytoplasmic production of YhbO enzyme, IPTG inducible 2) pET22b-yhbO, a plasmid designed for periplasmic production of YhbO enzyme, IPTG inducible and 3) pET28b empty as a negative control.

BL21(DE3) strains were grown in 100 mLTB (37 °C, 200 rpm) ; after induction with 0,1 mM IPTG followed by a protein production phase at 20°C overnight, catalysts were harvested by centrifugation at 4000 rpm for 10 minutes. For each strain, half of the cells were resuspended in 25 mL of water (these cells constituted the 'free biocatalysts'), the other half in 25 mL of 0,1 % glutaraldehyde solution for 1h at room temperature (these cells constituted the 'immobilized or crosslinked biocatalysts'). The harvested cells were washed with 25 mL PBS 1X and recovered by centrifugation.

The reaction consisting of 67 mg dcw catalyst/ml in a 5 ml solution of glyoxal (250 mM) and HEPES pH 7,5 (500 mM), was carried out at 25°C, 200 rpm.

The bioproduction process was monitored with high performance chromatography (HPLC) by withdrawing 0,5 mL samples at fixed time intervals. Glyoxalase activity is identical whereas the glyoxalase is located in the periplasmic or cytoplasmic space **(****Figure 7****),** free catalysts have identical specific activities, equal to or greater than 42,6 µmol glycolate.min⁻¹.g⁻¹dcw catalyst. The treatment using glutaraldehyde seems impacting the activity however about 75% of the activity remained: the reaction is still highly efficient and complete in 1h.

These results demonstrated that the glyoxalase activity and production of glycolate is identical whereas the glyoxalase is located in the periplasmic or cytoplasmic space, with even better results with glyoxalase is the periplasmic space. The immobilization of the biocatalysts seems slowing down the reaction and/or impacting the activity of the glyoxalase, but the maximum level of production of glycolate is obtained after 1 hour of treatment.

### 2.5 - Biocatalyst recycling

Two BL21(DE3) strains were produced as disclosed in example 1.2carrying respectively the plasmids 1) pET28b-yhbO, a plasmid design for cytoplasmic production of YhbO enzyme, IPTG inductible or 2) pET22b-yhbO, a plasmid designed for periplasmic production of YhbO enzyme, IPTG inductible were cultivated as described in the Example 2.4.

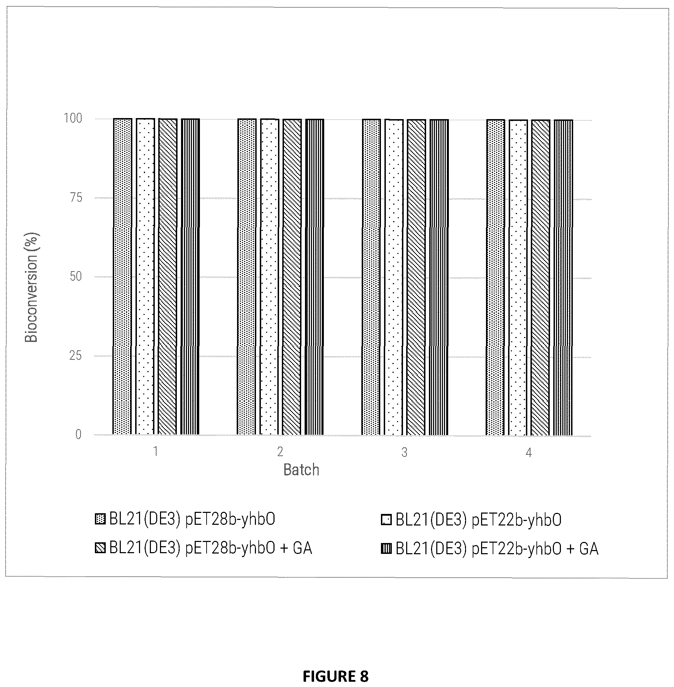

The two strains were cross-linked using glutaraldehyde as described in the Exemple 2.4. To investigate the effect of recycling on the degree of glyoxal conversion, biotransformation reaction were carried out both with free cells and cross-linked cells in batch mode. After 4 cycles, a complete conversion of glyoxal was observed in less than 2 hours for the six types of catalyst **(****Figure 8****).**

These results demonstrated that the biocatalysts can be recycled 2 to 3 times (4 cycles as above) without loosing their catalysis efficacy, as we obtained a complete conversion of glyoxal in less than 2 hours. The immobilization is advantageous for harvesting the biocatalyst at the end of a cycle and re-use it in a further cycle, either successively or in a delayed time.

### 2.6 Preparation of Carrageenan beads with E.coli BL21(DE3)/pET22b-yhbo and E.coli BL21(DE3)/pET28b-yhb0 respectively

Two catalysts were produced as disclosed in example 1.2 : 1) *E.coli* BL21(DE3)/pET22b-yhbO producing the glyoxalase III from *E.coli* in the periplasmic space and 2) *E.coli* BL21(DE3)/pET28b-yhbO producing the enzyme in the cytoplasmic space.

Both catalysts were immobilized using carrageenan. Stains were grown on TB, at 37°C, 200 rpm until OD600nm reached 0,6 where 0,1 mM IPTG were added to the media to induce YhbO expression at 20°C overnight. Harvested cells were resuspended in water, diluted twice in a solution of carrageenan 2,5%.

The suspension was maintened at 60°C in a water bath and poured drop by drop using a syringe into a solution KCL 0,3 M at 4°C. The reaction consisting of a mixture of all the beads (beads of carrageenan encapsulating the biocatalysts) formed into a 5 mL solution containing 250 mM glyoxal and 500 mM HEPES pH7,5, was carried out at 30°C 200 rpm. For both catalysts 100% conversion was achieved after 3h.

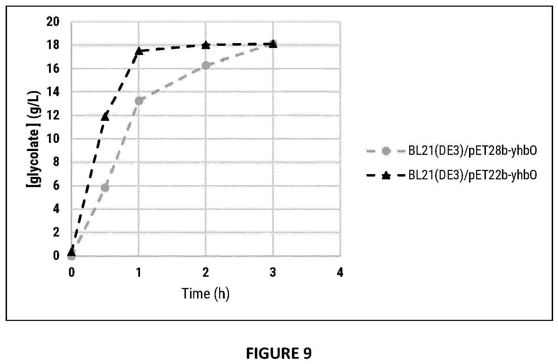

BL21(DE3)/pET22b-yhbO immobilized cells showed a specific activity of 60,7 µmol glycolate.min⁻¹.g⁻¹dcw catalyst, whereas BL21(DE3)/pET28b-yhbO immobilized cells has an activity of 51,8 µmol glycolate.min⁻¹.g⁻¹dcw catalyst **(****Figure 9****).**

The periplasmic secretion of the glyoxalase III is advantageous when cells are encapsulated (in carrageenan beads in the present example), certainly due to a better transport of molecules across the periplasm.

### 2.7 Preparation of glutaraldehyde (GA)/ polyethylenimine (PEI)-Crosslinked BL21(DE3)/pET22b-yhbO transformant expressing glyoxalase III from E.coli

BL21(DE3)/pET22b-yhbO strain was used to compare different treatment to stabilize glyoxalase activity, using polyethylenimine (PEI) to flocculate the cells and glutaraldehyde to crosslink them in order to provide a reaction product which can be used in a biocatalytic process multiple times.

Cells were produced as described in Exemple 2.4. A volume equivalent to 435 mg of dcw cells was centrifuged at 4000 rpm, 10 min. The harvested cells were washed with 50 mL PBS 1X and recovered by centrifugation. The different tested conditions are presented in the **table 6.**

**Table 6: Treatment conditions of BL21(DE3)/pET22b-yhbO strain using glutaraldehyde (GA) and/or polyethylenimine (PEI).**

| **Free cells** | **GA** | **GA + PEI** | **PEI + GA +PEI** |
|---|---|---|---|
| +25 mL H2O | + 25 mL H2O | + 25 mL H2O | + 75 mL H2O |
| | + GA (0,1% final) | + GA (0,1% final) | + PEI (0,25% final) |
| 1 hour at room temperature | | | |
| | | + 50 mL H2O | + GA (0,1% final) |
| | | + PEI (0,25% final) | + PEI (0,5% final) |
| 1 hour at room temperature | | | |
| Centrifugation at 4000 rpm, 10min | | Buchner filtration at 0,2µm + PBS 1X | |
| Washing 25 mL de PBS 1X | | Powder recovery | |
| Centrifugation at 4000 rpm 10min | | | |

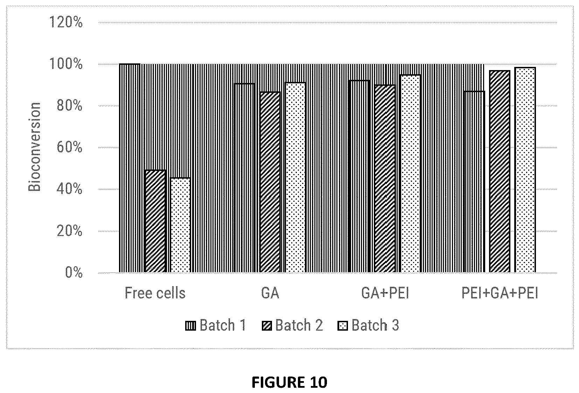

The reaction consisting of 90 mg/ml of free or treated cells in a 5 ml solution of glyoxal (250 mM) and HEPES pH 7,5 (50 mM), was carried out at 30°C, 200 rpm. The bioproduction process was monitored with high performance chromatography (HPLC) by withdrawing 0,5 mL samples at fixed time intervals. The pH was measured in each sample and, if necessary, re-adjusted in the reaction to pH 7.5 using 3 M NaOH. After 18h, cells were harvested and reuse in a fresh mixture containing glyoxal (250 mM) and HEPES pH 7,5 (50 mM). Three reactions in batch mode were carried oout, the bioconversion droped by 50 % from the second reuse for the free cells **(****Figure 10****).** The difference with the Exemple 2.5 can be explained by a lower concentration of HEPES buffer, cells were exposed to acidic pH between samples. Treated cells appeared to be protected from pH variations by GA and PEI, their catalytic efficiency remained stable around 90%. The stabilization of microorganisms using a cationic flocculating agent and a crosslinking agent is suitable for optimized glyoxal bioconversion.

### Example 7: Glyoxal bioconversion to glycolic acid in methanol

BL21(DE3) pET28b-yhbO strain was prepared as described in the Example 1.2. The reaction consisting of 67 mg dcw catalyst in a 1 ml methanol solution containing glyoxal (200 mM) was carried out at 25°C, 200 rpm. Reaction was monitored with high performance chromatography (HPLC) by withdrawing 0,2 mL samples after 3h. A total conversion of glyoxal to glycolic acid was observed, demonstrating the production of glycolic acid in organic solvant.

All these results demonstrated that the use of whole cells of transformed microorganisms as biocatalysts, such as E. coli strains (e.g BL21(DE3) strains) transformed with plasmids comprising YhbO gene with/without signal sequence and constitutive/inducible promoter, in a biochemical production according to the invention, permits to produce rapidly (few hours) glycolic acid from glyoxal with high yield and with quantity and volume productivity exceeding the performance of all published fermentation processes.

These biocatalysts may be used as free biocatalysts, or advantageously as immobilized biocatalysts, either crosslinked with glutaraldehyde (GA) and/or polyethylenimine (PEI), or encapsulated in beads of carrageenan, for facilitating their recycling and reuse in multiple reactions (from 2 to 4 cycles).

Finally, the use of biocatalysts expressing the glyoxalase in the periplasmic space of the said microorganisms offers several advantages in addition to the production of active proteins properly fold in an oxidizing environment. Small molecular solutes such as glyoxal and glycolic acid can freely equilibrate between exterior solution and the periplasm, unlike the cytoplasmic exchanges that can be tightly regulated throught the membrane active tranporters. Substrate is easily accessible for the periplasmic enzymes, product can instantly diffuse in the external media for an optimal reaction rate. Finally, the separation of the periplasm from the metabolic complexity of the cytoplasmic environment is advantageous for bioproduction : in this subcellular compartment side reactions are limited leading to few by-product production and high production yields.

### REFERENCES

- Abdallah, J., Caldas, T., Kthiri, F., Kern, R., & Richarme, G. (2007). YhbO protects cells against multiple stresses. Journal of Bacteriology, 189 (24), 9140-9144.
- BASF. (2016). Glyoxal More Sustainable Solutions for Your Business . 4.
- Chung, CT, Niemela, SL, & Miller, RH (1989). One-step preparation of competent Escherichia coli: transformation and storage of bacterial cells in the same solution. Proceedings of the National Academy of Sciences , 86 (7), 2172-2175.
- Ehrmann M. (2007). « The Periplasm - ASM Press, Washington DC »
- He, YC, Xu, JH, Su, JH, & Zhou, L. (2010). Bioproduction of Glycolic acid from glycolonitrile with a new bacterial isolate of alcaligenes sp. ECU0401. Applied Biochemistry and Biotechnology, 160 (5), 1428-1440. https://doi.org/10.1007/s12010-009-8607-y
- KATAOKA, M., SASAKI, M., HIDALGO, A.-RGD, NAKANO, M., & SHIMIZU, S. (2001). Glycolic Acid Production Using Ethylene Glycol-Oxidizing Microorganisms. In Bioscience, Biotechnology, and Biochemistry (Vol. 65, Issue 10, pp. 2265-2270). https://doi.org/10.1271/bbb.65.2265
- Lee, C., Lee, J., Lee, JY, & Park, C. (2015). Characterization of the Escherichia coli yajl, yhbo and elbb glyoxalases. FEMS Microbiology Letters , 363 (3), 1-7. https://doi.org/10.1093/femsle/fnv239
- Panova, A., Mersinger, LJ, Liu, Q., Foo, T., Roe, DC, Spillan, WL, Sigmund, AE, Ben-Bassat, A., Wagner, LW, O'Keefe, DP, Wu, S., Petrillo, KL, Payne, MS, Breske, ST, Gallagher, FG, & DiCosimo, R. (2007). Chemoenzymatic Synthesis of Glycolic Acid. Advanced Synthesis & Catalysis, 349 (8-9), 1462-1474. https://doi.org/10.1002/adsc.200700061
- Wei, G., Yang, X., Gan, T., Zhou, W., Lin, J., & Wei, D. (2009). High cell density fermentation of Gluconobacter oxydans DSM 2003 for glycolic acid production. Journal of Industrial Microbiology and Biotechnology, 36 (8), 1029-1034. https://doi.org/10.1007/s10295-009-0584-1

## Claims

1. A method for the biochemical production of glycolic acid or its derivatives comprising a step of culturing a whole cell biocatalyst comprising or consisting of modified microorganisms cells producing an enzyme converting glyoxal into glycolic acid, in a suitable culture medium comprising glyoxal as substrate, and optionally recovering glycolic acid from the culture medium.

2. The method according to claim 1, wherein the enzyme converting glyoxal into glycolic acid is produced into the cytoplasmic space or alternatively into the periplasmic space of the microorganisms cells, preferably into the periplasmic space of the microorganisms cells.

3. The method according to claim 1 or claim 2, wherein the enzyme converting glyoxal substrate into glycolic acid is a glyoxalase having a catalytic triad Cys-His-Asp/Glu, in particular a glyoxalase III (GLYIII) comprising advantageously an amino acid sequence having the conserved hallmark DJ-1_Pfpl domain, preferably the glyoxalase III encoded by YhbO gene from E. Coli (SEQ ID NO:1) or an amino acid sequence having at least 80% identity with SEQ ID NO:1.

4. The method according to anyone of claims 1 to 3, wherein the microorganism is a gram-negative strain selected in the group consisting of Enterobacteriaceae, Alcaligenaceae, Vibrionaceae and Pseudomonadaceae, in particular Enterobacteriaceae, preferably Enterobacteriaceae belonging to the Salmonella, Yersinia or Escherichia genus, more preferably Escherichia genus, and even more preferably *Escherichia coli.*

5. The method according to anyone of claims 1 to 4, wherein the microorganisms cells are modified by transformation with an expression cassette being on a circular DNA (plasmid) or integrated into the microorganism genome.

6. The method according to claim 5, wherein the expression cassette comprises a gene encoding a glyoxalase as defined in claim 3, under the control of a constitutive promoter or an inductible promoter.

7. The method according to claim 5 or 6, wherein the expression cassette also comprises a secretion signal gene for addressing the glyoxalase into the periplasmic space, in particular selected in the group consisting of dsbA, EOX, lamB, MgIB, MmAp, ompC, ompT, sufl, SfmC, STII, tolB, torA, torT, glll, malE, ompA, pelB, phoA, nlpA, preferably pelB.

8. The method according to anyone of claims 1 to 7, wherein the modified microorganisms cells are immobilized, in particular on agar or carrageenan, or alternatively the microorganisms cells are crosslinked, in particular with glutaraldehyde and/or polyethylene imine (PEI), or are both immobilized and crosslinked.

9. The method according to claim 8, wherein it further comprises a step of recycling the modified microorganisms cells producing an enzyme converting glyoxal into glycolic acid, at the end of the bioconversion reaction.

10. A whole cell biocatalyst comprising or constituting of modified microorganisms cells producing a enzyme able to convert glyoxal into glycolic acid, as defined in anyone of claims 3 to 6.

11. The whole cell biocatalyst according to claim 10, wherein the biocatalyst is immobilized on agar or carraghenaan or crosslinked with glutaraldehyde and/or polyéthylène imine.

12. A method of producing a whole cell biocatalyst as defined in claim 10, comprising the steps of :
a) Transforming the microorganisms cells by a plasmid comprising an expression cassette comprising a gene encoding glyoxalase GLYIII under the control of a constitutive or inducible promoter, and comprising optionally a secretion signal gene for addressing the glyoxalase into the periplasmic space; or alternatively a') engineering the microorganisms cells by integrating the said expression cassette into their genome;
b) Cultivating the modified cells in an adapted culture medium for the expression of glyoxalase ;
c) Separating the cells from the supernatant by centrifugation ; and
d) Optionally drying the cells and storing them at 4°C or -20°C or -80°C.

13. A method of producing a glycolic acid derivative or a product obtained by a reaction using glycolic acid as substrate, comprising at least a step of the method for the biochemical production of glycolic acid as defined in anyone of claims 1 to 9.

14. An expression cassette comprising a nucleotidic sequence encoding a glyoxalase as defined in claim 3 and optionally a secretion signal gene encoding a signal peptide as defined in claim 7 for adressing the said glyoxalase into the periplasmic space of a microorganism cell.

15. A nucleotide sequence encoding the expression cassette according to the claim 14.

16. A vector comprising a nucleotide sequence according to the claim 15.
